Europäisches Patentamt

European Patent Office

Office européen des brevets

(11) Publication number: 0 378 827
A1

# EUROPEAN PATENT APPLICATION

(21) Application number: 89123378.5

(51) Int. Cl.5: A61K 37/02

(22) Date of filing: 18.12.89

(30) Priority: 21.12.88 US 287812

(43) Date of publication of application:
25.07.90 Bulletin 90/30

(84) Designated Contracting States:
GR

(71) Applicant: SCHERING CORPORATION
2000 Galloping Hill Road
Kenilworth New Jersey 07033(US)

(72) Inventor: Black, Hugh E.
83 Ridge Road
Sparta New Jersey 07871(US)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Use of interleukin-4 for lowering blood glucose levels and/or effecting weight reduction.

(57) A method for lowering the blood-serum glucose in a mammal and/or effecting weight reduction by administering IL-4 is disclosed as well as the use of IL-4 for the manufacture of a medicament for use in lowering blood-serum glucose levels and/or effecting weight loss.

EP 0 378 827 A1

## USE OF INTERLEUKIN-4 FOR LOWERING BLOOD GLUCOSE LEVELS AND/OR EFFECTING WEIGHT REDUCTION

### BACKGROUND OF THE INVENTION

This invention relates to the lowering of blood glucose levels and/or effecting weight reduction in mammals by administering an effective amount of interleukin-4 (IL-4).

Interleukin-4 (IL-4) is a lymphokine (stimulator of the immune system) that has a broad range of immune cell stimulation as described in Banchereau et al., *Lymphokine Res.* Vol. 6, No. 1: U135 (1987); Yokoto et al., *Proc. Natl. Acad. Sci. USA*, 83: 5894-5898 (1986); Lee at al., *Proc. Natl. Acad. Sci. USA*, 83: 2061-2065 (1986); Coffman et al., *J. Immunol.* 136: 949-954 (1986); Sanderson et al., *Proc. Natl. Acad. Sci. USA*, 83: 437-440 (1986); Grabstein et al., *J. Exp. Med.*, 163: 1405-1413 (1985); and Vitetta et al., *J. Exp. Med.* 162: 1726-1731 (1985). During its early development IL-4 has also been referred to as B-cell growth factor (BCGF) [Butler et a., *J. Immunol.*, 133: 251-255 (1984)(human BCGF); and Farrar et al., *J. Immunol.*, 131: 1838-184 (1983)(mouse BCGF)] and B-Cell stimulatory factor 1 (BSF-1) [Ohara et al., *J. Immunol.*, 135: 2518-2523 (1985)]. The clarification and designation of the name interleukin-4 was finally proposed and adopted in 1986 [Sanderson et al., *Proc. Natl. Acad. Sci. USA*, 83: 437-440(1986)].

### SUMMARY OF THE INVENTION

The method of this invention involves administering to mammals a serum glucose lowering or a weight reducing effective amount of IL-4. Preferably, the IL-4 is administed to mammals diagnosed as having elevated blood serum glucose levels (hyperglycemia). The invention also relates to the use of IL-4 for the manufacture of a medicament for use in lowering blood-serum glucose levels and/or effecting weight reduction.

Preferably, the IL-4 employed is derived from a human source. Preferably the dosage form is one suitable for administration by intravenous injection or intravenous infusion and administration will suitable be in an amount of about 0.5 to about 600 micrograms of IL-4 per kilogram of body weight per day. Preferably, the IL-4 is administered in an amount of about 0.5 to about 125 micrograms of IL-4 per kilogram of body weight per day, and most preferably about 0.5 to about 30 micrograms of IL-4 per kilogram of body weight per day.

### DESCRIPTION OF THE DRAWING

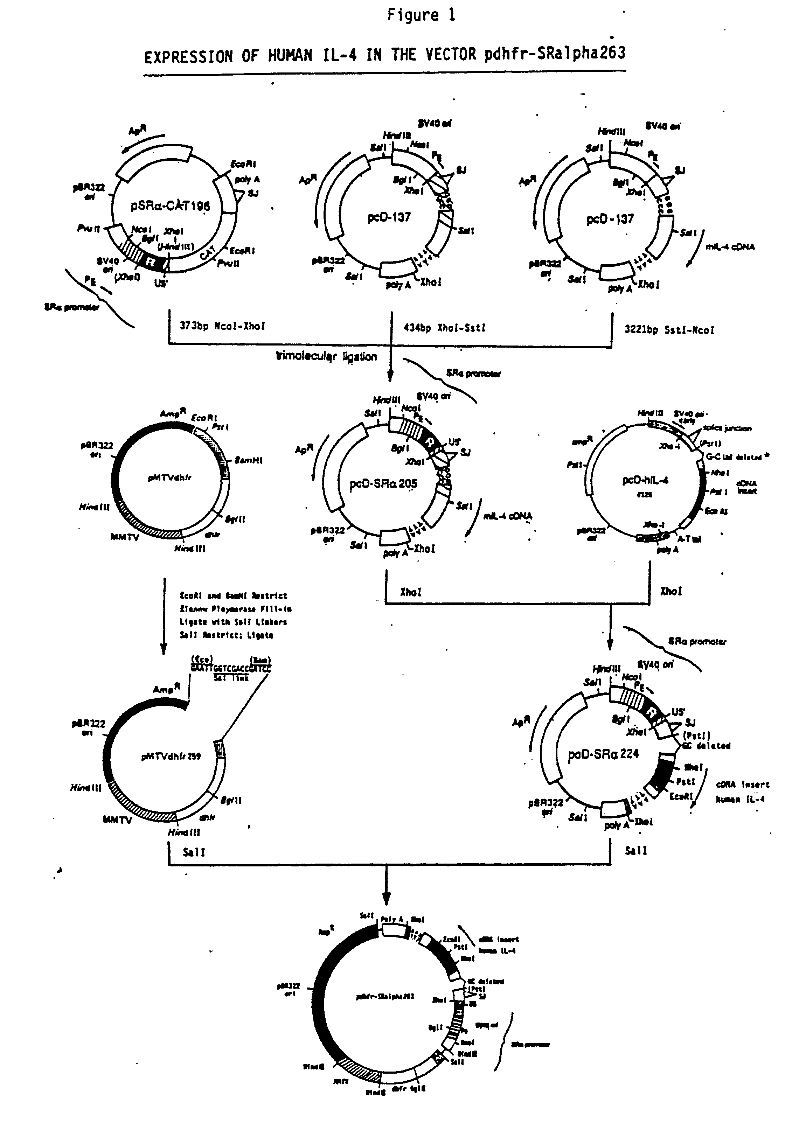

Figure 1. Construction map for the expression of human IL-4 in the vector pdhfr-SRalpha263.

### DETAILED DESCRIPTION OF THE INVENTION

The invention provides a method for lowering blood glucose levels in a mammal, e.g., mammals with hyperglycemia, by administering to said mammal a serum glucose lowering effective amount of IL-4 and/or a method for reducing weight in a mammal by administering a weight reducing effective amount of IL-4 to said mammal.

The invention also provides for the manufacture of a medicament for use in lowering blood-serum glucose and/or effecting weight reduction.

Any suitable IL-4 may be employed in the present invention. Complementary DNAs (cDNAs) for IL-4 have recently been cloned and sequenced by a number of laboratories, e.g. Yokoto et al., *Proc. Natl. Acad. Sci. USA*, 83: 5894-5898 (1986) (human); Lee at al., *Proc. Natl. Acad. Sci. USA*, 83: 2061-2065 (1986)-(mouse); Noma et al., *Nature* 319: 640-646 (1986)(mouse); and Genzyme Corporation, Boston, Massachusetts (human and mouse). Moreover, non-recombinant IL-4 has been purified from various culture supernatants, e.g. Sanderson et al., *Proc. Natl. Acad. Sci. USA*, 83: 437-440 (1986)(mouse); Grabstein et al., *J. Exp. Med.*, 163: 1405-1413 (1985)(mouse); Ohara et al.,*J. Immunol.*, 135: 2518-2523 (1985)(mouse BSF-1);

Butler et al., *J. Immunol.*, 133: 251-255 (1984)(human BCGF); and Farrar et al., *J. Immunol.*, 131: 1838-1842 (1983)(mouse BCGF). The disclosures of all the above articles are incorporated herein by reference for their teachings of DNA and amino acid sequences and of methods of obtaining suitable IL-4 materials for use in the present invention.

Preferably, the IL-4 used in the present invention will be a human IL-4, and most preferably it will be the human version with the sequence described in Yokoto et al., *Proc. Natl. Acad. Sci. USA,* 83: 5894-5898 (1986) and PCT Patent Application No. 87/02990 published May 21, 1987 that is expressed in (U.S Patent Application No. 079,666, filed July 29, 1987) and isolated from (U.S. Patent Application No. 194,799, filed July 12, 1988) E. coli. The disclosures of the above articles, PCT Application and U.S. Patent Application are hereby incorporated herein by reference.

According to this invention, mammals are administered a serum glucose lowering or weight reducing effective amount of an IL-4. A serum glucose lowering effective amount is defined as any amount that will significantly lower the glucose level, with a lowering of glucose by at least 5 percent considered significant. A weight reducing effective amount is defined as any amount that will result in a reduction in total body weight at a rate which is consistent with the continued health of the patient as defined by parameters established by the attending physician, with weight losses of 0.5 to 2 pounds per week considered about average for an adult human. From about 0.5 to about 600 micrograms of IL-4, preferably human IL-4 (hIL-4), per kilogram of body weight per day is preferably administered. More preferably, mammals are administered about 0.5 to about 125 micrograms of hIL-4 per kilogram of body weight per day, and most preferably mammals are administered about 0.5 to about 30 micrograms of hIL-4 per kilogram of body weight per day.

The amount, frequency and period of administration will vary depending upon factors such as severity of the glucose or weight elevation, age of the patient, nutrition, etc. Usually, the administration will be daily initially and it may continue periodically during the patient's lifetime. Dosage amount and frequency may be determined during initial screenings of glucose and weight levels and the magnitude of the effect of IL-4 upon the lowering of the glucose or weight levels. Dosage will be aimed to lower the glucose level to an acceptable level of about 100 milligrams of glucose per deciliter of blood serum or to lower weight to that which is considered normal for a given individual.

To complement the glucose lowering effect of IL-4, it may be useful to administer it in conjunction with other pharmaceutically active compounds. For example, it can be combined with insulin and the like, which are commercially available from a number of sources known to those skilled in the art.

For use in a regime of weight loss, it can be administered in conjunction with other weight reducing agents and/or anorectic agents such as tumor necrosis factor (TNF; see PCT Patent Application No. 86/06280, published November 6, 1986); phenylpropanolamine, dextroamphetamine, methamphetamine, benzephetamine, phentermine, diethylproprion, phenmetrazine, phendimetrazine or other known weight reducing agents (for example, see Blaser et al., *Eur. J. Pharm.* 134: 97-103 (1987) and The Pharmacological Basis of Therapeutics, 4th edition, Edited by Goodman and Gilman, The Macmillan Co., New York, 1970, pp. 518-523). These references are hereby incorporated by reference to illustrate examples of other agents that can used in combination with IL-4 in certain embodiments of the present invention. The specific glucose lowering agents and weight reducing agents mentioned above are merely examples of such agents known to those skilled in the art that can be used in the practice of the present invention.

Administration of the dose can be intravenous, nasal, parenteral, oral, subcutaneous, intramuscular, topical, transdermal or any other acceptable method. The IL-4 could be administered in any number of conventional dosage forms. Parenteral preparations include sterile solutions or suspensions. Inhalation administration can be in the form of a nasal or oral spray, or by insufflation. Topical dosage forms can be creams, ointments, lotions, transdermal devices (e.g., of the conventional resevoir or matrix patch type) and the like.

The formulations and pharmaceutical compositions contemplated by the above dosage forms can be prepared with conventional pharmaceutically acceptable excipients and additives, using conventional techniques.

Presently, the IL-4 is administered via the intravenous route. The solutions to be administered may be reconstituted lypholized powders and they may additionally contain preservatives, buffers, dispersants, etc. Preferably, IL-4 is reconstituted with 10 millimolar citrate buffer and preservative-free sterile water with the maximum concentration not to exceed 1500 micrograms per milliliter and administered by continuous intravenous infusion or by intravenous injection. For continuous infusion, the daily dose can be added to 5 ml of normal saline and the solution infused by mechanical pump or by gravity.

The effect of IL-4 on lowering the serum glucose levels in mammals can be determined by the following test protocol.

Cynomolgus monkeys (Macaca fascicularis) are administered human IL-4, which is obtained from Chinese Hamster Ovary (CHO) cells as described below. The route of administering the human IL-4 is by intravenous injection in the saphenous vein for about 15 seconds and dosing occurrs daily for four weeks. Blood samples are taken at the times indicated in the following table by femoral venipuncture following an overnight fast. Blood samples are taken at two times prior to dosing with the human IL-4 (-2 and -1 weeks prior to initial IL-4 dosing) and control animals (receiving IL-4 doses of 0.0 mg/kg/day in Table 1) are maintained that never received any dose of the human IL-4. The blood samples are checked for blood glucose levels using an enzymatic (hexokinase) DACOS analyzer which is commercially available from Coulter Electronics, Inc., 600 West 20 Street, Hialeah, FL 33010.

Taking the averages for the various doses in Table 1 resulted in percent lowering of glucose levels of 51% (after 3 weeks) and 57% (after 4 weeks) at 0.025 mg/kg dose, 51% (after 3 weeks) and 53% (after 4 weeks) at 0.125 mg/kg dose, and 34% (after 2 and 3 weeks) at 0.600 mg/kg dose. Weight loss was also observed and the amount of weight loss appeared to be dose dependent.

## Construction of the Human IL-4 Expression Plasmid pdhfr-SRalpha263

The construction of plasmids pSRα-CAT196, pcD137, pcD-SRα205, pcDhIL-4 clone 125 and pcD-SRα224 have been described (Takebe et al., *Molecular and Cellular Biology*, 8: 466-477 (1988); and Yokoto et al., *Proc. Natl. Acad. Sci. USA*, 83: 5894-5898 (1986)). As shown in Figure 1, plasmid pcD-SRα-205 was constructed by the trimolecular ligation of the 373bp Ncol-Xhol SRα promoter fragment from pSRα-CAT196, the 434bp Xhol-SstI splice junction (SJ) and 5' murine IL-4 (mIL-4) fragment from pcD137 and the 3221bp SstI-Ncol fragment, also from pcD137, containing the 3' murine IL-4 cDNA, SV40 polyadenylation region and the pBR322 derived plasmid backbone containing the origin of replication and ampicillin resistance gene. The G-C tail was deleted from pcD-hIL-4 clone 46 (Yokoto et al., *Proc. Natl. Acad. Sci. USA*, 83: 5894-5898)) as follows. The Okayama-Berg plasmid pL1 (Okayma and Berg, *Molecular and Cellular Biology*, 3: 280-289(1983)) was restricted with PstI and the four nucleotide overhang removed by the 3'-5' exonuclease activity of T4 polymerase. BglII linkers were ligated to the flush DNA ends followed by restriction with BglII and HindIII. The HindIII BglII fragment containing the SV40 sequence of pL1 was isolated and inserted into BglII/HindIII restricted pcD-MCGF (Yokoto et al., *Proc. Natl. Acad. Sci. USA*, 81: 1070-1074 (1984)) to yield intermediate plasmid 101. The purified 3Ilbp Pst fragment from plasmid pcD-hIL-4 clone 46 was restricted with Sau3A-I which releases a 163bp fragment with overhangs compatible with BglII. The 162bp fragment was ligated to BglII restricted p101 to yield intermediate 112. The HindIII/NheI fragment of p112 containing $SV_{40}$ and human IL-4 cDNA sequences was ligated to HindIII/NheI restricted clone 46 DNA to produce pcD-hIL-4 clone 125 containing an $SV_{40}$ early promoter, SV40 splice junction and complete human IL-4 cDNA with the G-C tail deleted. Plasmid pcD-Srα224 was constructed by replacing the small Xhol fragment of pcD-SRα205 (containing the SJ and mIL4 cDNA) with the small Xhol fragment of pcDhIL-4 clone 125 containing the SJ and HIL-4 cDNA with G-C tail deleted as described above.

A Sall site was introduced into pMTVdhfr (Lee et al., *Nature*, 294: 228-232 (1981)) by EcoRI/BamHI restriction, Klenow polymerase fill in of the overhang and ligation to an octanucleotide sall linker as shown in Figure 1. Plasmid pMTVdhfr259, then, lacks restriction sites for EcoRI and BamHI and the region between the two is replaced with a Sall linker. The Sall fragment of pcD-SRα224 containing the Srα promoter, SV40 SJ, human IL 4 cDNA and SV40 polyadenylation signals was inserted into the unique Sall site of pMTVdhfr259. The final human IL-4 expression plasmid, pdhfr-SRalpha263 contains the following elements, counterclockwise from the Sall site:
1. Ampicillin resistance gene and origin of replication from pBR322.
2. MMTV LTR driven dhfr expression unit from pMTVdhfr.
3. SRalpha promoter.
4. SV40 derived splice junction.
5. Human IL-4 cDNA.
6. SV40 derived polyadenylation signal.

The human IL-4 cDNA sequence present in the vector is the same as in pcD-HIL-4 clone 46 given in Yokoto et al., *Proc. Natl. Acad. Sci. USA*, 83: 5894-5898 (1986).

## DHFR Gene Amplification and Selection of IL-4 SI Line

Chinese hamster ovary cell mutants deficient in dihydrofolate reductase activity (CHO-dhfr⁻) are widely used for overproduction of various recombinant proteins. (Kaufman et al., *Molecular and Cellular Biology,* 5: 1750-1759 (1985)). CHO-dhfr⁻ mutant cells have an auxotrophic requirement for hypoxhantine, thymidine and glycine. Expression vectors incorporating a dhfr marker may be used to complement this mutation; selection is achieved by growing cells in the absence of the required media cofactors described above. Gene amplification (increase in copy number up to 1000X) may be accomplished by growing cells in increasing concentration of the folate analog methotrexate (MTX). The amplification of the integrated recombinant dhfr locus in the genome results in a concomitant increase in copy number of the expression unit for the gene of interest. (Ringhold et al., *J Mol. Applied Genetics,* 1: 165-175 (1981); and Kaufman et al., *EMBO J.,* 6: 187-193 (1987)).

The plasmid DNA having the coding sequence for dhfr and human IL-4 (pdhfr-SRα263) was constructed as described above. Transfection of pdhfr-SRα263 into DXB-II CHO-dhgr⁻ cell line was carried out by the calcium phosphate precipitation method. (Graham and Van der Eb, Virology, 52: 546 (1978)) Transformants were selected in a selection medium (DMEM, Dulbecco's Modified Eagle's Medium) that lacks hypoxhantine and thymidine. A clone designated 3B12 was chosen for the first cycle of amplification. The 3B12 clone was cultured in α-MEM medium (Eagle's minimum essential medium) containing 40 nM MTX until resistant clones were selected. A clone designated 3B12-A26 was used for further amplification with 1 μm MTX. After the second cycle of drug selection, a clone designated 3B12-A26-19 was chosen for further development. This clone was adapted to growth in a suspension mode with 10% NU Serum™ V and a subclone designated IL-4 SI was selected for the large scale propagation.

Culture Preparation

In order to prepare a Master Cell Bank (MCB), two original 100ml spinner flasks containing the IL-4 SI cells were used. The cells were carried through two additional growth medium exchanges and grown in 100 ml spinner flasks (growth medium is basal medium plus 0 to 10% serum, e.g., NU Serum™ V). Cells from each flask were collected, washed, resuspended in 10 ml of freezing medium, pooled and aseptically dispensed in about 2.0 ml sterile cell storage vials (freezing medium is basal medium plus 20% serum, e.g., NU Serum™ V plus 10% dimethylsulfoxide). The vials were slowly frozen at -70°C and stored in liquid nitrogen.

The cells from three frozen vials were thawed and propagated by suspension in growth medium for 4 to 6 generations in spinner flasks of increasing volume from 100 ml up to 3 liters. Cells were collected by centrifugation, washed, and resuspended in freezing medium. The cell suspension was aseptically dispensed in about 2.0 ml sterile cell storage vials. The vials were slowly frozen at -70°C and stored in liquid nitrogen to constitute the Master Cell Bank (MCB).

A Master Working Cell Bank (MWCB) was prepared from the MCB by thawing 1 to 3 vials of the MCB and propagating the cells in T-flasks and in suspension for 4 to 6 generations in increasing volumes up to 3 liters. Cells were collected, washed, resuspended in freezing medium and aliquoted and frozen as described for the MCB. The MWCB was stored in liquid nitrogen as well.

IL-4 production was carried out in bioreactors of 50 to 200 liters in volume, To start production, one frozen vial from the MWCB was thawed and inoculated into a T-75 flask. From incubation until cell concentration reaches 100% confluency, cells were trypsinized and inoculated into two T-75 flasks (optionally, a T-160 flask can be used). These flasks were again incubated until 100% confluency and the trypsinized cells were used to inoculate a 100 ml spinner flask.

The 100 ml spinner flask was incubated until adequate cell growth was obtained and was used as inoculum for a 250 ml spinner flask. A similar step was repeated in a 1 liter and a 3 liter flask and a 10 to 20 liter bioreactor. Cells from the 10 to 20 liter reactor were used as inoculum for a 50 to 100 liter reactor. This reactor is initially grown batchwise and upon achieving adequate cell concentration, a continuous media perfusion was initiated.

The media used for growth and continuous perfusion was modified Iscove's medium, which may be supplemented with up to 10% (E.g., NU Serum™ V). No methoxtrexate was used throughout the production process.

The fermentation stages were carried out under sterile conditions and in closed systems. The key fermentation parameters such as temperature, pH, agitation and aeration were monitored and controlled as appropriate throughout the growth and continuous perfusion stages. Aseptic samples were taken periodically to measure pH, cell density and to check for sterility (absence of bacteria and fungi).

Upon collection of an adequate volume of conditioned media (perfusate), the broth was filtered to remove any cells that may be present, and concentrated via ultrafiltration. The concentrate, which contains crude CHO IL-4, was forwarded to the final purification stages.

Purification of IL-4 from the crude CHO IL-4 concentrate was carried out by performing a cation exchage chromatography on a sulphonate column (e.g., S-Sepharose). This step was typically repeated. Selected pooled fractions from the sulphonate column were then forwarded to a chelate chromatography step (e.g., cobalt-chelate Sepharose). The selected pooled chelate fractions were then diafiltered and concentrated via membrane filtration. The concentration was chromatographed in a gel filtration column (e.g., HR S-200). The pooled fractions which constitute the purified bulk IL-4 were then filtered and stored at -20° C or lower.

The bulk IL-4 was then prepared for injection by thawing and diluted with sterilized water and/or 10 mm citrate buffer.

The above references are hereby incorporated by reference for their relevant teachings of materials and methods used in the construction of the CHO expression system for hIL-4.

## Table 1
## Glucose Lowering Effect of IL-4 in Monkeys

| Animal No. | IL-4 Dose (mg/kg/day) | Week of Dosing[a] | | | |
|---|---|---|---|---|---|
| | | -2 | -1 | 3 | 4 |
| 2(M) | 0.0 | 151 | 116 | 96 | 91 |
| 3(M) | 0.0 | 154 | 116 | 78 | 84 |
| 26(M) | 0.0 | 83 | 84 | 71 | 78 |
| 4(F) | 0.0 | 147 | 169 | 82 | 105 |
| 6(F) | 0.0 | 100 | 111 | 64 | 86 |
| 28(F) | 0.0 | 109 | 148 | 88 | 119 |
| 7(M) | 0.025 | 116 | 86 | 32 | 39 |
| 8(M) | 0.025 | 99 | 82 | 30 | 29 |
| 9(M) | 0.025 | 98 | 88 | 55 | 82 |
| 10(M) | 0.025 | 135 | 125 | 46 | 46 |
| 11(M) | 0.025 | 104 | 96 | 31 | 20 |
| 12(M) | 0.025 | 126 | 127 | 40 | 26 |
| 13(M) | 0.125 | 88 | 102 | 30 | 39 |
| 14(M) | 0.125 | 143 | 93 | 36 | 32 |
| 15(M) | 0.125 | 103 | 98 | 29 | 55 |
| 17(M) | 0.125 | 101 | 111 | 51 | 91 |
| 18(M) | 0.125 | 92 | 88 | 44 | 18 |
| 29(M) | 0.125 | 98 | 101 | 46 | 31 |
| 19(M) | 0.600 | 117 | 117 | 18[b] | ND |
| 21(M) | 0.600 | 149 | 110 | 75[b] | ND |
| 27(M) | 0.600 | 125 | 127 | ND | ND |
| 22(F) | 0.600 | 87 | 98 | 65[b] | ND |
| 23(F) | 0.600 | 89 | 105 | 63[b] | ND |
| 30(F) | 0.600 | 95 | 108 | 43 | ND |

M = males

F = females

ND = not determined

[a] glucose amounts expressed as milligrams per deciliter
[b] measurements taken at 2 weeks after dosing

## Claims

1. A method of treatment for lowering the blood-serum glucose level in a mammal and/or effecting weight reduction which comprises administering to said mammal an effective amount of IL-4.

2. A method as claimed in claim 1, wherein the mammal is a human.

3. A method as claimed in claim 1 or claim 2, wherein the IL-4 administered in human IL-4.

4. A method as claimed in any one of the preceding claims wherein the IL-4 is administered in an amount of about 0.5 micrograms to about 600 micrograms per kilogram of body weight per dose.

5. A method as claimed in claim 4 wherien the dose is administered daily.

6. A method as claimed in any one of the preceding claims wherein administration is effected by intravenous infusion or intravenous injection.

7. A method as claimed in any one of the preceding claims wherein the IL-4 is administered in combination with another glucose-lowering agent or weight-reducing agent.

8. The use of IL-4 for the manufacture of a medicament for use in lowering blood-serum glucose levels and/or effecting weight reduction.

9. The use of IL-4 for lowering blood-serum glucose levels and/or effecting weight reduction.

10. The use as claimed in claim 8 or claim 9, wherein the IL-4 is human IL-4.

# Figure 1

## EXPRESSION OF HUMAN IL-4 IN THE VECTOR pdhfr-SRalpha263

European Patent Office

**PARTIAL EUROPEAN SEARCH REPORT**
which under Rule 45 of the European Patent Convention
shall be considered, for the purposes of subsequent
proceedings, as the European search report

Application number

EP 89 12 3378

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | EP-A-0 230 107 (SCHERING BIOTECH CORP.) | | A 61 K 37/02 |
| A | WO-A-88 04 667 (IMMUNEX CORP.) | | |

---------

**TECHNICAL FIELDS SEARCHED (Int. Cl.4)**

C 12 P

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 8,10

Claims searched incompletely:

Claims not searched: 1-7,9

Reason for the limitation of the search:

Method for treatment of the human
or animal body by surgery or therapy
(See art. 52(4) of the European
Patent Convention)

| Place of search | Date of completion of the search | Examiner |
|---|---|---|
| The Hague | 03-04-1990 | REMPP |